(19) Europäisches Patentamt
European Patent Office
Office européen des brevets

(11) EP 4 699 664 A2

(12) **EUROPEAN PATENT APPLICATION**

(43) Date of publication:
**25.02.2026 Bulletin 2026/09**

(21) Application number: **25220515.8**

(22) Date of filing: **12.05.2021**

(51) International Patent Classification (IPC):
***A61Q 5/02*** *(2006.01)*

(52) Cooperative Patent Classification (CPC):
**A61Q 5/02; A61K 8/604; A61K 8/737**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.05.2020 US 202063025445 P**

(62) Document number(s) of the earlier application(s) in accordance with Art. 76 EPC:
**21730358.5 / 4 149 416**

(71) Applicant: **The Procter & Gamble Company**
**Cincinnati, OH 45202 (US)**

(72) Inventors:
- **BALLHAUS, Lauren Ellizabeth**
**Cincinnati, 45202 (US)**
- **HUTTON III, Howard David**
**Cincinnati, 45202 (US)**
- **MAZZEO, Nicole Marie**
**Cincinnati, 45202 (US)**
- **ZHAO, Jean Jianqun**
**Cincinnati, 45202 (US)**

(74) Representative: **P&G Patent Germany**
**Procter & Gamble Service GmbH**
**Sulzbacher Straße 40**
**65824 Schwalbach am Taunus (DE)**

Remarks:
This application was filed on 03-12-2025 as a divisional application to the application mentioned under INID code 62.

(54) **SHAMPOO COMPOSITION COMPRISING DECYL GLUCOSIDE AND A CATIONIC GUAR**

(57) A shampoo composition comprising a nonionic surfactant, and a cationic guar having a weight average molecular weight of from about 800,000g/mol to about 5,000,000g/mol, and a charge density of from about 0.1 meg/g to about 2.5 meg/g. The shampoo is phase stable and delivers a consumer desirable in use viscosity while still providing cleaning, moisture and slip feel hair benefits.

EP 4 699 664 A2

## Description

FIELD OF THE INVENTION

The present disclosure generally relates to mild shampoo compositions free of ionic surfactants which clean and condition with a high molecular weight cationic guar polymer for increased consumer conditioning benefits.

BACKGROUND OF THE INVENTION

Human hair becomes soiled due to contact with the surrounding environment and from sebum secreted by the scalp. Soiled hair has a dirty feel and exhibits an unattractive appearance. Application and washing of the soiled hair with a shampoo composition can restore hair to a clean and attractive appearance by removing oil and other soils from the hair. Known shampoo compositions typically remove oil and soil from hair through inclusion of anionic surfactants. Shampoos including anionic surfactants, such as sodium lauryl sulfate and sodium laureth sulfate, however, can exhibit a number of undesirable characteristics such as poor quality hair feel as well as hair and skin dryness after washing. Shampoos including anionic surfactants also face poor consumer acceptance as a consequence of this harshness. Cationic polymers are commonly used in anionic surfactant cleansing compositions to provide moisturization and wet detangle to hair. Non-ionic surfactants are known for mildness on skin but are also known to be difficult to use in combination with charged polymers. Additionally, non-ionic surfactant systems are typically thin (low viscosity) and usually require a thickening polymer to increase viscosity to prevent solution dripping off of consumers hands prior to application to hair. Commonly used thickening polymer for non-ionic surfactant systems include non-ionic polymers, such as guar gum. However, these non-ionic polymers do not provide consumer desired hair benefits such as conditioning. It is desirable to have a shampoo composition that cleans without the use of anionic surfactants and also has good in use physical properties while delivering the desired hair benefits. Surprisingly it has been found that a shampoo composition comprising a nonionic surfactant, and a cationic guar having a weight average molecular weight of from about 800,000g/mol to about 5,000,000g/mol and a charge density of from about 0.1 meg/g to about 2.5 meg/g, is phase stable and delivers consumer desired in use viscosity as well as cleaning, moisture and slip feel hair benefits (conditioning benefits).

SUMMARY OF THE INVENTION

A shampoo composition comprising from about 8% to about 35% of decyl glucoside; less than 1% of a surfactant selected from sodium alkyl sulfate, sodium cocoyl isethionate, sodium lauroyl sarcosinate, cocamidopropyl betaine, sodium lauroamphoacetate, cetyltrimethylammonium chloride, behenyltrimethylammonium chloride and the mixture thereof; from about 0.4% to about 1.2% cationic guar polymer having a weight average molecular weight of from about 800,000/mol to about 5,000,000 g/mol and a charge density of from about 0.1 meg/g to about 2.5meg/g; wherein the shampoo composition has a viscosity from about 300cps to 8,000cps.

DETAILED DESCRIPTION OF THE INVENTION

While the specification concludes with claims which particularly point out and distinctly claim the invention, it is believed the present disclosure will be better understood from the following description.

Definitions

In all embodiments of the present disclosure, all percentages are by weight of the total composition, unless specifically stated otherwise. All ratios are weight ratios, unless specifically stated otherwise. All ranges are inclusive and combinable. The number of significant digits conveys neither a limitation on the indicated amounts nor on the accuracy of the measurements. All numerical amounts are understood to be modified by the word "about" unless otherwise specifically indicated. Unless otherwise indicated, all measurements are understood to be made at about 25 °C and at ambient conditions, where "ambient conditions" means conditions under about one atmosphere of pressure and at about 50% relative humidity. All such weights as they pertain to listed ingredients are based on the active level and do not include carriers or by-products that may be included in commercially available materials, unless otherwise specified.

As used herein, "molecular weight" or "Molecular weight" refers to the weight average molecular weight unless otherwise stated. Molecular weight is measured using industry standard method, gel permeation chromatography ("GPC").

The term "charge density," as used herein, refers to the ratio of the number of positive charges on a polymer to the molecular weight of said polymer.

The term "comprising," as used herein, means that other steps and other ingredients which do not affect the end

result can be added. This term encompasses the terms "consisting of" and "consisting essentially of." The compositions and methods/processes of the present disclosure can comprise, consist of, and consist essentially of the elements and limitations of the invention described herein, as well as any of the additional or optional ingredients, components, steps, or limitations described herein.

The term "polymer," as used herein, includes materials whether made by polymerization of one type of monomer or made by two (*i.e.,* copolymers) or more types of monomers.

The term "suitable for application to human hair," as used herein, means that the personal care compositions or components thereof, are acceptable for use in contact with human hair and the scalp and skin without undue toxicity, incompatibility, instability, allergic response, and the like.

The term "water soluble," as used herein, means that the material is soluble in water. The material can be soluble at 25 °C at a concentration of 0.1% by weight of the water solvent, at 1% by weight of the water solvent, at 5% by weight of the water solvent, and at 15% or more by weight of the water solvent.

The terms "sulfate free" and "substantially free of sulfates" means essentially free of sulfate-containing compounds except as otherwise incidentally incorporated as minor components.

The term "sulfated surfactants" means surfactants which contain a sulfate group. The term "substantially free of sulfated surfactants" means essentially free of surfactants containing a sulfate group except as otherwise incidentally incorporated as minor components.

Shampoo Composition

The shampoo composition as described herein provides consumer desired moisture conditioning feel, and the shampoo composition is stable and has a viscosity which delivers a good in use experience. The shampoo composition comprises a nonionic surfactant, such as decyl glucoside, from about 8 wt% to about 35 wt% non-ionic surfactant, and a cationic guar having a relatively high MW from about 800,000g/mol to about 5,000,000g/mol weight average molecular weight, and with a CD from about 0.1 meg/g to about 2.5meg/g weight average charge density. This product remains phase stable and good viscosity and continues to provide cleaning, moisture and slip feel hair benefits.

Additionally, the shampoo comprises a low amount of ionic surfactant, including sodium alkyl sulfate, sodium cocoyl isethionate, sodium lauroyl sarcosinate, cocamidopropyl betaine, sodium lauroamphoacetate, cetyltrimethylammonium chloride, behenyltrimethylammonium chloride and the mixtures thereof. The amount of ionic surfactant is from about 0 wt% to about 1 wt%, from about 0 wt% to about 0.5 wt%, from about 0.1 wt% to about 0.2 wt%, alternatively from about 0 wt% to about 0.3 wt%.

The shampoo composition has a viscosity of from about 300cps to 8,000cps. The viscosity can also be from about 500 cps to about 7000 cps, from about 500 to about 6000 cps, from about 800 to about 4000 cps or any combination thereof.

Non-Ionic Surfactant

The shampoo composition comprises from about 8% to about 35% of a non-ionic surfactant. The non-ionic surfactant can be a polyglucoside selected from the group consisting of decyl glucoside, lauryl glucoside, octyl glucoside, caprylyl glucoside, caprylyl/capryl glucoside, undecyl glucoside, coco glucoside, myristyl glucoside, hexadeyl glucoside, octadecyl glucoside, arachidyl glucoside, cetyl glucoside, cetearyl glucoside, isostearyl glucoside, and mixtures thereof. The non-ionic surfactant can be decyl glucoside. The shampoo composition can comprise about from about 8 wt% to about 30 wt%, from about 8 wt% to about 25 wt%, from about 7 wt% to about 20 wt%, of the non-ionic surfactant.

The non-ionic surfactant can be an alkyl polyglucoside having the structure,

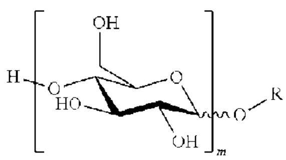

in which "R" is an alkyl or alkenyl group having 8 to 20 carbons, and "m" is degree of polymerization of from 1 to 5. Alternatively, the R is from 8 to 16 carbons, and alternatively wherein the R is from 8 to 12 carbons.

The non-ionic surfactant can be a decyl glucoside having the structure :

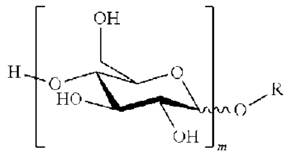

in which "R" is an alkyl or alkenyl group having 10 carbons, and "m" degree of polymerization is 1.

Cationic Guar

The shampoo composition comprises from about 0.4 wt% to about 1.2 wt% guar gum, alternatively from about 0.8 wt% to about 1.0 wt%, from about 0.4 wt% to about 1.0 wt%, and/or from about 1.0 wt% to about 1.2 wt%, or any combination thereof. Guar gum for use in preparing these guar gum derivatives is typically obtained as a naturally occurring material from the seeds of the guar plant. The guar molecule itself is a straight chain mannan, which is branched at regular intervals with single membered galactose units on alternative mannose units. The mannose units are linked to each other by means of β (1-4) glycosidic linkages. The galactose branching arises by way of an α (1-6) linkage. Cationic derivatives of the guar gums are obtained by reaction between the hydroxyl groups of the polygalactomannan and reactive quaternary ammonium compounds. The degree of substitution of the cationic groups onto the guar structure should be sufficient to provide the requisite cationic charge density described above.

The cationic guar polymer can have a weight average molecular weight of from about 800,000g/mol to about 5,000,000g/mol, from about 800,000g/mol to about 3,000,000g/mol, from about 900,000g/mol to about 3,000,000g/mol, from about 800,000g/mol to about 2,500,000g/mol, and/or from about 900,000g/mol to about 2,500,000g/mol.

The cationic guar polymer can have a charge density of from about 0.1 meq/g to about 2.5 meg/g, from about 0.2 meq/g to about 2.0 meg/g, from about 0.3 meq/g to about 1.9 meg/g, from about 0.4 meq/g to about 1.8 meg/g, from about 0.5 meq/g to about 1.7 meg/g, from about 0.5 meq/g to about 1.5 meq/g, from about 0.5 meq/g to about 1.4 meg/g, from about 0.6 meq/g to about 1.3, and/or from about 0.7 meq/g to about 1.25 meg/g.

The cationic guar polymer may be formed from quaternary ammonium compounds. The quaternary ammonium compounds for forming the cationic guar polymer can conform to the general formula 1:

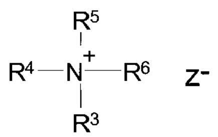

wherein where $R^3$, $R^4$ and $R^5$ are methyl or ethyl groups; $R^6$ is either an epoxyalkyl group of the general formula 2:

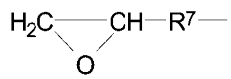

or $R^6$ is a halohydrin group of the general formula 3:

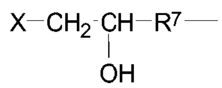

wherein $R^7$ is a $C_1$ to $C_3$ alkylene; X is chlorine or bromine, and Z is an anion such as Cl-, Br-, I- or $HSO_4$-.

The cationic guar polymer can conform to the general formula 4:

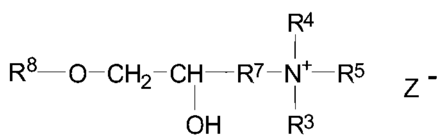

wherein $R^8$ is guar gum; and wherein $R^4$, $R^5$, $R^6$ and $R^7$ are as defined above; and wherein Z is a halogen. The cationic guar polymer can conform to Formula 5:

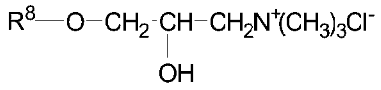

The cationic guar polymer may be formed from quaternary ammonium compounds. Suitable cationic guar polymers include cationic guar gum derivatives, such as guar hydroxypropyltrimonium chloride. The cationic guar polymer is a guar hydroxypropyltrimonium chloride. Specific examples of guar hydroxypropyltrimonium chlorides include the Jaguar® series commercially available from Solvay , for example Jaguar Excel has a charge density of 0.75 meq/g and a weight average molecular weight of g/mol, Jaguar C17 has a charge density of 0.68 meq/g and a weight average molecular weight of 2,200,00g/mol, Jaguar C135 and Jaguar C145 which have a charge density of 0.75 meq/g and a weight average molecular weight of 2,200,00g/mol, n-Hance series commercially available from Ashland, for example n-Hance 3196 and n-Hance 3205 which have a charge density of 0.75 meq/g and a weight average molecular weight of 1,650,000g/mol, n-Hance 3215 has a charge density of 1.1meq/g and a weight average molecular weight of 1,600,000g/mol, n-Hance 3198 has a charge density of 0.68 meq/g and a weight average molecular weight of 1,500,000g/mol, n-Hance CG17 has a charge density of about 1.0meq/g and a weight average molecular weight of about 1,600,000 g/mol, and Dehyquart Guar series commercially available from BASF, for example Dehyquart Guar HP has a charge density of 1.0 meq/g and a weight average molecular weight of 2,000,000 g/mol, Dehyquart Guar N has a charge density of 0.8 meq/g and a weight average molecular weight of 2,250,000 g/mol, and Dehyquart Guar TC has a charge density of 1.0 meq/g and a weight average molecular weight of 1,350,000 g/mol .

Other suitable guar hydroxypropyltrimonium chloride are: Hi-Care 1000, which has a charge density of about 0.7 meq/g and a weight average Molecular weight of about 600,000 g/mole is available from Rhodia; BF-13, which is a borate (boron) free guar of charge density of about 1.1 meq/g and weight average molecular weight of about 800,000 and BF-17, which is a borate (boron) free guar of charge density of about 1.7 meq/g and weight average molecular weight of about 800,000 both available from Ashland.

Liquid Carrier for the Shampoo Composition

The shampoo composition also includes a liquid carrier. Inclusion of an appropriate quantity of a liquid carrier can facilitate the formation of a shampoo composition having an appropriate viscosity and rheology. A shampoo composition can include, by weight of the composition, about 60% to about 95% of a liquid carrier, about 65% to about 92%, about 70% to about 90% of a liquid carrier, and about 75% to about 90% of a liquid carrier.

A liquid carrier can be water or can be a miscible mixture of water and organic solvent. A liquid carrier can be water with minimal or no significant concentrations of organic solvent, except as otherwise incidentally incorporated into the composition as minor ingredients of other essential or optional components. Suitable organic solvents can include water solutions of lower alkyl alcohols and polyhydric alcohols. Useful lower alkyl alcohols include monohydric alcohols having 1 to 6 carbons, such as ethanol and isopropanol. Exemplary polyhydric alcohols include propylene glycol, hexylene glycol, glycerin, and propane diol.

Optional Components

As can be appreciated, shampoo compositions described herein can include a variety of optional components to tailor the properties and characteristics of the composition. As can be appreciated, suitable optional components are well known and can generally include any components which are physically and chemically compatible with the essential components of the shampoo compositions described herein. Optional components should not otherwise unduly impair product stability, aesthetics, or performance. Individual concentrations of optional components can generally range from about 0.001% to about 10%, by weight of a shampoo composition.

Suitable optional components which can be included in a shampoo composition can include natural ingredients such as, tea extracts, and natural antioxidants such as grape seed extracts, natural hair conditioning oils, such as safflower oil, jojoba oil, argan oil, and combinations thereof.

Suitable optional components which can be included in a shampoo composition can include co-surfactants, deposition aids, cationic polymers, conditioning agents (including hydrocarbon oils, fatty esters, silicones), anti-dandruff agents, suspending agents, viscosity modifiers, dyes, nonvolatile solvents or diluents (water soluble and insoluble), pearlescent aids, foam boosters, pediculocides, pH adjusting agents, perfumes, preservatives, chelants, proteins, skin active agents, sunscreens, UV absorbers, and vitamins.

**Silicone Emulsions**

The hair care composition can comprise from about 0% to about 10%, from about 0.1% to about 8%, from about

0.1% to about 5%, from about 0.1% to about 4%, from about 0.1% to about 3%, from about 0.1% to about 2%, from about 0.1% to about 1.5%, and/or from about 0.1% to about 1.2%, by weight, of one of more silicone polymers. The silicone polymer can be added into the hair care composition as an aqueous pre-emulsion. The silicone pre-emulsion can comprise one or more silicone polymers and an emulsifying system. The silicone polymer content in the silicone pre-emulsion can be from about 10%, by weight, to about 70%, by weight, or about 15%, by weight, to about 60%, by weight, or from about 18%, by weight, to about 50% by weight.

The silicone emulsion can have an average particle size of less than 500 nm, alternatively 300 nm, alternatively less than about 200 nm, and alternatively less than about 100 nm. The silicone emulsion can have an average particle size of from about 5 nm to about 500 nm, from about 10- nm to about 400 nm, and/or from about 20 nm to about 300 nm. The silicone emulsion can be in the form of a nano-emulsion.

The particle size of the one or more silicones may be measured by dynamic light scattering (DLS). A Malvern Zetasizer Nano ZEN3600 system using He-Ne laser 633nm may be used for the measurement at 25°C.

The autocorrelation function may be analyzed using the Zetasizer Software provided by Malvern Instruments, which determines the effective hydrodynamic radius, using the Stokes-Einstein equation:

$$D = \frac{k_B T}{6\pi\eta R}$$

wherein $k_B$ is the Boltzmann Constant, T is the absolute temperature, $\eta$ is the viscosity of the medium, D is the mean diffusion coefficient of the scattering species, and R is the hydrodynamic radius of particles.

Particle size (i.e. hydrodynamic radius) may be obtained by correlating the observed speckle pattern that arises due to Brownian motion and solving the Stokes-Einstein equation, which relates the particle size to the measured diffusion constant, as is known in the art.

For each sample, 3 measurements may be made and Z-average values may be reported as the particle size.

The one or more silicones may be in the form of a nanoemulsion. The nanoemulsion may comprise any silicone suitable for application to the skin and/or hair.

The one or more silicones may include in their molecular structure polar functional groups such as Si-OH (present in dimethiconols), primary amines, secondary amines, tertiary amines, and quaternary ammonium salts. The one or more silicones may be selected from the group consisting of aminosilicones, pendant quaternary ammonium silicones, terminal quaternary ammonium silicones, amino polyalkylene oxide silicones, quaternary ammonium polyalkylene oxide silicones, and amino morpholino silicones.

The one or more silicones may comprise:

(a) at least one aminosilicone corresponding to formula (V):

$$R'_aG_{3-a}\text{-}Si(OSiG_2)_n\text{-}(OSiG_bR'_{2-b})_m\text{-}O\text{-}SiG_{3-a}\text{-}R'_a \qquad (I)$$

in which:

G is chosen from a hydrogen atom, a phenyl group, OH group, and $C_1$-$C_8$ alkyl groups, for example methyl,
a is an integer ranging from 0 to 3, and in one embodiment a is 0,
b is chosen from 0 and 1, and in one embodiment b is 1,
m and n are numbers such that the sum (n+m) can range for example from 1 to 2 000, such as for example from 50 to 150, wherein n can be for example chosen from numbers ranging from 0 to 1 999, such as for example from 49 to 149, and wherein m can be chosen from numbers ranging for example from 1 to 2 000, such as for example from 1 to 10;
R' is a monovalent group of formula $-C_qH_{2q}L$ in which q is a number from 2 to 8 and L is an optionally quaternized amine group chosen from the groups:

$-NR''-CH_2-CH_2-N'(R^1)_2$,

$-N(R'')_2$,

$-N^+(R'')_3A^-$,

$-N^+H(R'')_2A^-$,

$-N^+H_2(R'')A^-$,

and

$-N(R'')-CH_2-CH_2-N^+R''H_2A^-$,

in which R" can be chosen from a hydrogen atom, phenyl groups, benzyl groups, and saturated monovalent hydrocarbon-based groups, such as for example an alkyl group comprising from 1 to 20 carbon atoms, and $A^-$ is chosen from halide ions such as, for example, fluoride, chloride, bromide and iodide.

The one or more silicones may include those corresponding to formula (1) wherein a = 0, G=methyl, m and n are numbers such that the sum (n+m) can range for example from 1 to 2 000, such as for example from 50 to 150, wherein n can be for example chosen from numbers ranging from 0 to 1 999, such as for example from 49 to 149, and wherein m can be chosen from numbers ranging for example from 1 to 2 000, such as for example from 1 to 10; and L is $-N(CH_3)_2$ or $-NH_2$, alternatively $-NH_2$.

Additional said at least one aminosilicone of the invention include:

(b) pendant quaternary ammonium silicones of formula (VII):

(VII)

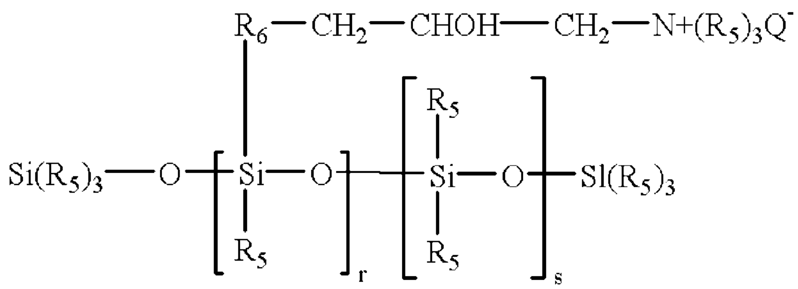

in which:

$R_5$ is chosen from monovalent hydrocarbon-based groups comprising from 1 to 18 carbon atoms, such as $C_1$-$C_{18}$ alkyl groups and $C_2$-$C_{18}$ alkenyl groups, for example methyl;
$R_6$ is chosen from divalent hydrocarbon-based groups, such as divalent $C_1$-$C_{18}$ alkylene groups and divalent $C_1$-$C_{18}$ alkylenoxy groups, for example $C_1$-$C_8$ alkylenoxy groups, wherein said $R_6$ is bonded to the Si by way of an SiC bond;
$Q^-$ is an anion that can be for example chosen from halide ions, such as chloride, and organic acid salts (such as acetate);
r is an average statistical value ranging from 2 to 20, such as from 2 to 8;
s is an average statistical value ranging from 20 to 200, such as from 20 to 50.

Such aminosilicones are described more particularly in U.S. Pat. No. 4,185,087, the disclosure of which is incorporated by reference herein.

A silicone which falls within this class is the silicone sold by the company Union Carbide under the name "Ucar Silicone ALE 56".

Further examples of said at least one aminosilicone include:

c) quaternary ammonium silicones of formula (VIIb):

(VIIb)

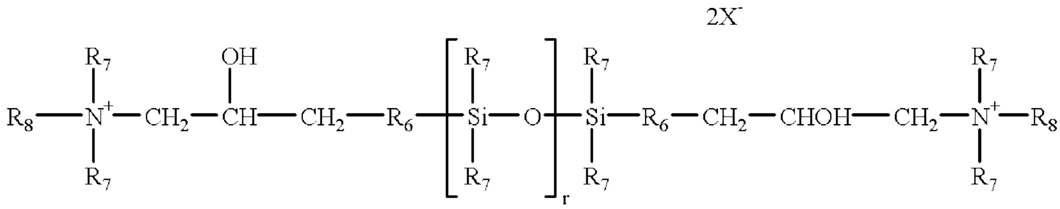

in which:

groups $R_7$, which may be identical or different, are each chosen from monovalent hydrocarbon-based groups comprising from 1 to 18 carbon atoms, such as $C_1$-$C_{18}$ alkyl groups, for example methyl, $C_2$-$C_{18}$ alkenyl groups, and rings comprising 5 or 6 carbon atoms;
$R_6$ is chosen from divalent hydrocarbon-based groups, such as divalent $C_1$-$C_{18}$ alkylene groups and divalent $C_1$-$C_{18}$alkylenoxy, for example $C_1$-$C_8$, group connected to the Si by an SiC bond;
$R_8$, which may be identical or different, represent a hydrogen atom, a monovalent hydrocarbon-based group comprising from 1 to 18 carbon atoms, and in particular a $C_1$-$C_{18}$ alkyl group, a $C_2$-$C_{18}$ alkenyl group or a group -$R_6$-$NHCOR_7$;
$X^-$ is an anion such as a halide ion, in particular chloride, or an organic acid salt (acetate, etc.);
r represents an average statistical value from 2 to 200 and in particular from 5 to 100.
Such silicones are described, for example, in application EP-A-0 530 974, the disclosure of which is incorporated by reference herein.
Silicones falling within this class are the silicones sold by the company Evonik under the names Abil Quat 3270, Abil Quat 3272, Abil Quat 3474 and Abil ME 45.
Further examples of said at least one aminosilicone include:

d) quaternary ammonium and polyalkylene oxide silicones
wherein the quaternary nitrogen groups are located in the polysiloxane backbone, at the termini, or both.
Such silicones are described in PCT Publication No. WO 2002/010257, the disclosure of which is incorporated by reference herein.
Silicones falling within this class are the silicones sold by the company Momentive under the names Silsoft Q.
(e) Aminofunctional silicones having morpholino groups of formula (V):

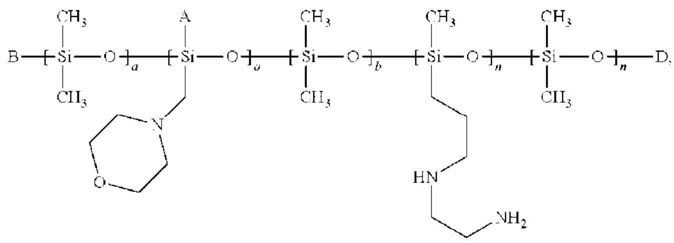

(V)

in which
A denotes a structural unit (I), (II), or (III) bound via -O-

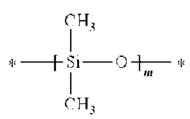

(I)

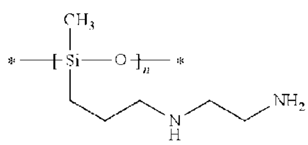

(II)

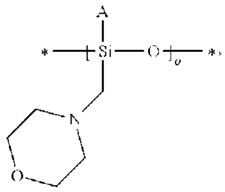

(III)

or an oligomeric or polymeric residue, bound via -O-, containing structural units of formulas (I), (II), or (III), or half of a connecting oxygen atom to a structural unit (III), or denotes -OH,

* denotes a bond to one of the structural units (I), (II), or (III), or denotes a terminal group B (Si-bound) or D (O-bound),
B denotes an -OH, $-O-Si(CH_3)_3$, $-O-Si(CH_3)_2OH$, $-O-Si(CH_3)_2OCH_3$ group, D denotes an -H, $-Si(CH_3)_3$, $-Si(CH_3)_2OH$, $-Si(CH_3)_2OCH_3$ group,
a, b, and c denote integers between 0 and 1000, with the provision that a+b+c>0,
m, n, and o denote integers between 1 and 1000.

Aminofunctional silicones of this kind bear the INCI name: Amodimethicone/Morpholinomethyl Silsesquioxane Copolymer. A particularly suitable amodimethicone is the product having the commercial name Wacker Belsil® ADM 8301E.
Examples of such silicones are available from the following suppliers:

*offered by the company Dow Corning:*Fluids: 2-8566, AP 6087, AP 6088, DC 8040 Fluid, fluid 8822A DC, DC 8803 & 8813 polymer, 7-6030, AP-8104, AP 8201;Emulsions: CE-8170 AF Micro Emulsion, 2-8177, 2-8194 Microemulsion, 9224 Emulsion, DC 1872 Emulsion, 939, 949, 959, DC 5-7113 Quat Microemulsion, DC 5-7070 Emulsion, DC CE-8810, CE 8401 Emulsion, CE 1619, Dow Corning Toray SS-3551, Dow Corning Toray SS-3552;
*offered by the company Wacker:*Wacker Belsil ADM 652, ADM 656, 1100, 1600, 1650 (fluids) ADM 6060 (linear amodimethicone) emulsion; ADM 6057 E (branched amodimethicone) emulsion; ADM 8020 VP (micro emulsion); SLM 28040 (micro emulsion); DM5500 emulsion;
*offered by the Company Momentive:*Silsoft 331, SF1708, SME 253 & 254 (emulsion), SM2125 (emulsion), SM 2658 (emulsion), Silsoft Q (emulsion)
*offered by the company Shin-Etsu:*KF-889, KF-867S, KF-8004, X-52-2265 (emulsion);
*offered by the Company Siltech Silicones:* Siltech E-2145, E-Siltech 2145-35;
*offered by the company Evonik Industries:* Abil T Quat 60th

Some non-limiting examples of aminosilicones include the compounds having the following INCI names: Silicone Quatemium-1, Silicone Quaternium-2, Silicone Quaternium-3, Silicone Quaternium-4, Silicone Quaternium-5, Silicone Quaternium-6, Silicone Quaternium-7, Silicone Quaternium-8, Silicone Quaternium-9, Silicone Quaternium-10, Silicone Quatemium-11, Silicone Quaternium-12, Silicone Quaternium-15, Silicone Quaternium-16, Silicone Quaternium-17, Silicone Quaternium-18, Silicone Quaternium-20, Silicone Quaternium-21, Silicone Quaternium-22, Quaternium-80, as well as Silicone Quaternium-2 Panthenol Succinate and Silicone Quaternium-16/Glycidyl Dimethicone Crosspolymer.
The aminosilicones can be supplied in the form of a nanoemulsion and include MEM 9049, MEM 8177, MEM 0959, MEM 8194, SME 253, and Silsoft Q.
The one or more silicones may include dimethicones, and/or dimethiconols. The dimethiconols are hydroxyl terminated dimethylsilicones represented by the general chemical formulas

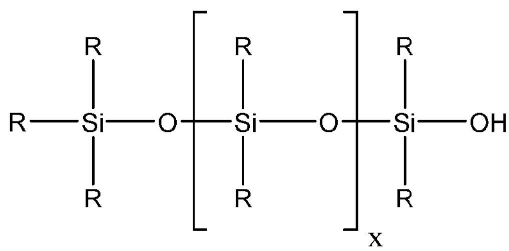

and

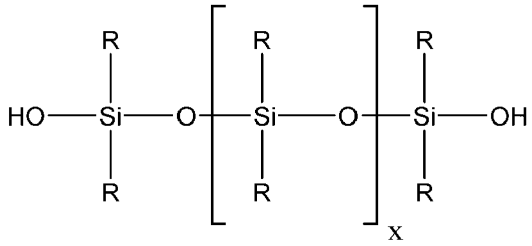

wherein R is an alkyl group (preferably R is methyl or ethyl, more preferably methyl) and x is an integer up to about 500, chosen to achieve the desired molecular weight. Commercial dimethiconols typically are sold as mixtures with dimethicone or cyclomethicone (e.g.,Dow Coming® 1401, 1402, and 1403 fluids).

According to another aspect of the silicone emulsion, the emulsion further includes an anionic surfactant that participates in providing high internal phase viscosity emulsions having particle sizes in the range from about 30 nm to about 10 microns. The anionic surfactant is selected from organic sulfonic acids. Most common sulfonic acids used in the present process are alkylaryl sulfonic acid; alkylaryl polyoxyethylene sulphonic acid; alkyl sulfonic acid; and alkyl polyoxyethylene sulfonic acid. General formulas of the sulfonic acids are as shown below:

R16C6H4SO3H (I)
R16C6H4O(C2H4O)mSO3H (II)
R16SO3H (III)
R16O(C2H4O)mSO3H (IV)

Where R16, which may differ, is a monovalent hydrocarbon radical having at least 6 carbon atoms. Non-limiting examples of R16 include hexyl, octyl, decyl, dodecyl, cetyl, stearyl, myristyl, and oleyl. 'm' is an integer from 1 to 25. Exemplary anionic surfactants include but are not limited to octylbenzene sulfonic acid; dodecylbenzene sulfonic acid; cetylbenzene sulfonic acid; alpha-octyl sulfonic acid; alpha-dodecyl sulfonic acid; alpha-cetyl sulfonic acid; polyoxyethylene octylbenzene sulfonic acid; polyoxyethylene dodecylbenzene sulfonic acid; polyoxyethylene cetylbenzene sulfonic acid; polyoxyethylene octyl sulfonic acid; polyoxyethylene dodecyl sulfonic acid; and polyoxyethylene cetyl sulfonic acid. Generally, 1 to 15% anionic surfactant is used in the emulsion process. For example, 3-10% anionic surfactant can be used to obtain an optimum result. The silicone emulsion may further include an additional emulsifier together with the anionic surfactant, which along with the controlled temperature of emulsification and polymerization, facilitates making the emulsion in a simple and faster 5 way. Non-ionic emulsifiers having a hydrophilic lipophilic balance (HLB) value of 10 to 19 are suitable and include polyoxyalkylene alkyl ether, polyoxyalkylene alkylphenyl ethers and polyoxyalkylene sorbitan esters. Some useful emulsifiers having an HLB value of 10 to 19 include, but are not limited to, polyethylene glycol octyl ether; polyethylene glycol lauryl ether; polyethylene glycol tridecyl ether; polyethylene glycol cetyl ether; polyethylene glycol stearyl ether; polyethylene glycol nonylphenyl ether; polyethylene glycol dodecylphenyl ether; polyethylene glycol cetylphenyl ether; polyethylene glycol stearylphenyl ether; polyethylene glycol sorbitan mono stearate; and polyethylene glycol sorbitan mono oleate.

Non-Silicone Conditioning Agents

The conditioning agent of the hair care compositions described herein may also comprise at least one organic conditioning agents, either alone or in combination with other conditioning agents, such as the silicones described above. Non-limiting examples of organic conditioning agents are described below.

a. Hydrocarbon Oils
Suitable organic conditioning agents for use as conditioning agents in hair care compositions include, but are not limited to, hydrocarbon oils having at least about 10 carbon atoms, such as cyclic hydrocarbons, straight chain aliphatic hydrocarbons (saturated or unsaturated), and branched chain aliphatic hydrocarbons (saturated or unsaturated), including polymers and mixtures thereof. Straight chain hydrocarbon oils can be from about $C_{12}$ to about $C_{19}$. Branched chain hydrocarbon oils, including hydrocarbon polymers, typically will contain more than 19 carbon atoms.
b. Polyolefins
Organic conditioning oils for use in the hair care compositions described herein also include liquid polyolefins, including liquid poly-$\alpha$-olefins and/or hydrogenated liquid poly-$\alpha$-olefins. Polyolefins for use herein are prepared by polymerization of $C_4$ to about $C_{14}$ olefenic monomers, alternatively from about $C_6$ to about $C_{12}$.
c. Fatty Esters

Other suitable organic conditioning agents for use as a conditioning agent in the hair care compositions described herein include fatty esters having at least 10 carbon atoms. These fatty esters include esters with hydrocarbyl chains derived from fatty acids or alcohols. The hydrocarbyl radicals of the fatty esters hereof may include or have covalently bonded thereto other compatible functionalities, such as amides and alkoxy moieties (e.g., ethoxy or ether linkages, etc.). Other oligomeric or polymeric esters, prepared from unsaturated glyceryl esters can also be used as conditioning materials.

d. Fluorinated Conditioning Compounds

Fluorinated compounds suitable for delivering conditioning to hair as organic conditioning agents include perfluoropolyethers, perfluorinated olefins, fluorine based specialty polymers that may be in a fluid or elastomer form similar to the silicone fluids previously described, and perfluorinated dimethicones.

e. Fatty Alcohols

Other suitable organic conditioning oils for use in the hair care compositions described herein include, but are not limited to, fatty alcohols having at least about 10 carbon atoms, about 10 to about 22 carbon atoms, and alternatively from about 12 to about 16 carbon atoms.

f. Alkyl Glucosides and Alkyl Glucoside Derivatives

Suitable organic conditioning oils for use in the hair care compositions described herein include, but are not limited to, alkyl glucosides and alkyl glucoside derivatives. Specific non-limiting examples of suitable alkyl glucosides and alkyl glucoside derivatives include Glucam E-10, Glucam E-20, Glucam P-10, and Glucquat 125 commercially available from Amerchol.

g. Polyethylene Glycols

Additional compounds useful herein as conditioning agents include polyethylene glycols and polypropylene glycols having a molecular weight of up to about 2,000,000 such as those with CTFA names PEG-200, PEG-400, PEG-600, PEG-1000, PEG-2M, PEG-7M, PEG-14M, PEG-45M and mixtures thereof.

2. Emulsifiers

A variety of anionic and nonionic emulsifiers can be used in the hair care compositions. The anionic and nonionic emulsifiers can be either monomeric or polymeric in nature. Monomeric examples include, by way of illustrating and not limitation, alkyl ethoxylates, alkyl sulfates, soaps, and fatty esters and their derivatives. Polymeric examples include, by way of illustrating and not limitation, polyacrylates , polyethylene glycols, and block copolymers and their derivatives. Naturally occurring emulsifiers such as lanolins, lecithin and lignin and their derivatives are also non-limiting examples of useful emulsifiers.

Anti-Dandruff Actives

A shampoo composition can also contain an anti-dandruff agent. Suitable anti-dandruff agents can include pyridinethione salts, azoles, selenium sulfide, particulate sulfur, and mixtures thereof. Such anti-dandruff particulate should be physically and chemically compatible with the essential components of the composition, and should not otherwise unduly impair product stability, aesthetics or performance. A shampoo composition can include a cationic polymer to enhance deposition of an anti-dandruff active.

a. Pyridinethione salts

An anti-dandruff agent can be a pyridinethione particulate such as a 1-hydroxy-2-pyridinethione salt. The concentration of pyridinethione anti-dandruff particulates can range from about 0.1% to about 4%, about 0.1% to about 3%, and from about 0.3% to about 2% by weight of the composition. Suitable pyridinethione salts include those formed from heavy metals such as zinc, tin, cadmium, magnesium, aluminum and zirconium, particularly suitable is the zinc salt of 1-hydroxy-2-pyridinethione (known as "zinc pyridinethione" or "ZPT"), 1-hydroxy-2-pyridinethione salts in platelet particle form, wherein the particles have an average size of up to about 20 $\mu$, up to about 5 $\mu$, up to about 2.5 $\mu$. Salts formed from other cations, such as sodium, can also be suitable. Pyridinethione anti-dandruff agents are further described in U.S. Patent No. 2,809,971; U.S. Patent No. 3,236,733; U.S. Patent No. 3,753,196; U.S. Patent No. 3,761,418; U.S. Patent No. 4,345,080; U.S. Patent No. 4,323,683; U.S. Patent No. 4,379,753; and U.S. Patent No. 4,470,982, each of which are incorporated herein by reference. It is contemplated that when ZPT is used as the anti-dandruff particulate, that the growth or re-growth of hair may be stimulated or regulated, or both, or that hair loss may be reduced or inhibited, or that hair may appear thicker or fuller.

b. Other Anti-Microbial Actives

In addition to the anti-dandruff active selected from polyvalent metal salts of pyrithione, a shampoo composition can further include one or more anti-fungal or anti-microbial actives in addition to the metal pyrithione salt actives. Suitable anti-microbial actives include coal tar, sulfur, whitfield's ointment, castellani's paint, aluminum chloride, gentian violet, octopirox (piroctone olamine), ciclopirox olamine, undecylenic acid and it's metal salts, potassium permanganate, selenium sulphide, sodium thiosulfate, propylene glycol, oil of bitter orange, urea preparations, griseofulvin, 8-hydroxyquinoline ciloquinol, thiobendazole, thiocarbamates, haloprogin, polyenes, hydroxypyridone, morpholine, benzylamine, allylamines (such as terbinafine), tea tree oil, clove leaf oil, coriander, palmarosa, berberine, thyme red, cinnamon oil, cinnamic aldehyde, citronellic acid, hinokitol, ichthyol pale, Sensiva SC-50, Elestab HP-100, azelaic acid, lyticase, iodopropynyl butylcarbamate (IPBC), isothiazalinones such as octyl isothiazalinone and azoles, and combinations thereof. Suitable anti-microbials can include itraconazole, ketoconazole, selenium sulphide and coal tar.

c. Soluble anti-dandruff agent

A suitable anti-microbial agent can be one material or a mixture selected from the groups consisting of: azoles, such as climbazole, ketoconazole, itraconazole, econazole, and elubiol; hydroxy pyridones, such as piroctone olamine, ciclopirox, rilopirox, and MEA-Hydroxyoctyloxypyridinone; kerolytic agents, such as salicylic acid and other hydroxy acids; strobilurins such as azoxystrobin and metal chelators such as 1,10-phenanthroline. Examples of azole anti-microbials can include imidazoles such as benzimidazole, benzothiazole, bifonazole, butaconazole nitrate, climbazole, clotrimazole, croconazole, eberconazole, econazole, elubiol, fenticonazole, fluconazole, flutimazole, isoconazole, ketoconazole, lanoconazole, metronidazole, miconazole, neticonazole, omoconazole, oxiconazole nitrate, sertaconazole, sulconazole nitrate, tioconazole, thiazole, and triazoles such as terconazole and itraconazole, and combinations thereof. When present in a shampoo composition, the soluble anti-microbial active can be included in an amount from about 0.01% to about 5%, from about 0.5% to about 6%, from about 0.1% to about 3%, from about 0.1% to about 9%, from about 0.1% to about 1.5%, from about 0.1% to about 2%, and more from about 0.3% to about 2%, by weight of the composition.

d. Selenium Sulfide

Selenium sulfide is a particulate anti-dandruff agent suitable for use as an anti-microbial composition when included at concentrations of about 0.1% to about 4%, by weight of the composition, from about 0.3% to about 2.5% by weight, and from about 0.5% to about 1.5% by weight. Selenium sulfide is generally regarded as a compound having one mole of selenium and two moles of sulfur, although it may also be a cyclic structure that conforms to the general formula $Se_xS_y$, wherein $x + y = 8$. Average particle diameters for the selenium sulfide are typically less than 15μm, as measured by forward laser light scattering device (e.g. Malvern 3600 instrument), less than 10 μm. Selenium sulfide compounds are described, for example, in U.S. Patent No. 2,694,668; U.S. Patent No. 3,152,046; U.S. Patent No. 4,089,945; and U.S. Patent No. 4,885,107, each of which are incorporated herein by reference.

e. Sulfur

Sulfur can also be used as a particulate anti-microbial/anti-dandruff agent. Effective concentrations of the particulate sulfur are typically from about 1% to about 4%, by weight of the composition, alternatively from about 2% to about 4%.

f. Keratolytic Agents

Keratolytic agents such as salicylic acid can also be included in a shampoo composition described herein.

g. Other

Additional anti-microbial actives can include extracts of melaleuca (tea tree), wintergreen (such as gaultheria procumbens leaf) and charcoal. As can be appreciated, shampoo compositions can also include combinations of anti-microbial actives. Suitable combinations can include octopirox and zinc pyrithione combinations, pine tar and sulfur combinations, salicylic acid and zinc pyrithione combinations, octopirox and climbasole combinations, and salicylic acid and octopirox combinations, and mixtures thereof.

Humectant

A shampoo composition can also include a humectant to lower the rate of water evaporation. Suitable humectants can include polyhydric alcohols, water soluble alkoxylated nonionic polymers, and mixtures thereof. The humectants, when included, can be used at levels by weight of the composition of from about 0.1% to about 20%, and from about 0.5% to about 5%.

Suitable polyhydric alcohols can include glycerin, sorbitol, propylene glycol, butylene glycol, hexylene glycol, ethoxylated glucose, 1, 2-hexane diol, hexanetriol, dipropylene glycol, erythritol, trehalose, diglycerin, xylitol, maltitol, maltose, glucose, fructose, sodium chondroitin sulfate, sodium hyaluronate, sodium adenosine phosphate, sodium lactate, pyrrolidone carbonate, glucosamine, cyclodextrin, and mixtures thereof.

Suitable water soluble alkoxylated nonionic polymers can include polyethylene glycols and polypropylene glycols having a molecular weight of up to about 1000 such as those with CTFA names PEG-200, PEG-400, PEG-600, PEG-1000, and mixtures thereof.

Other Optional Components

As can be appreciated, a shampoo composition can include still further optional components. For example, amino acids can be included. Suitable amino acids can include water soluble vitamins such as vitamins B1, B2, B6, B12, C, pantothenic acid, pantothenyl ethyl ether, panthenol, biotin, and their derivatives, water soluble amino acids such as asparagine, alanin, indole, glutamic acid and their salts, water insoluble vitamins such as vitamin A, D, E, and their derivatives, water insoluble amino acids such as tyrosine, tryptamine, and their salts.

A shampoo composition can include pigment materials such as inorganic, nitroso, monoazo, disazo, carotenoid, triphenyl methane, triaryl methane, xanthene, quinoline, oxazine, azine, anthraquinone, indigoid, thionindigoid, quinacridone, phthalocianine, botanical, natural colors, including: water soluble components such as those having C. I. Names. The compositions can also include antimicrobial agents which are useful as cosmetic biocides and antidandruff agents including: water soluble components such as piroctone olamine, water insoluble components such as 3,4,4'- trichlorocarbanilide (trichlosan), triclocarban and zinc pyrithione.

One or more stabilizers and preservatives can be included. For example, one or more of trihydroxystearin, ethylene glycol distearate, citric acid, sodium citrate dihydrate, a preservative such as kathon, sodium chloride, sodium benzoate, and ethylenediaminetetraacetic acid ("EDTA") can be included to improve the lifespan of a shampoo compositon.

Chelants can also be included to scavenge metal and reduce hair damage caused by exposure to UV radiation. Examples of suitable chelants can include histidine and N,N' ethylenediamine disuccinic acid ("EDDS").

Method of Use

The shampoo compositions described herein can be used in a conventional manner for cleansing and conditioning of hair or skin. Generally, a method of treating hair or skin can include applying the shampoo composition to the hair or skin. For example, an effective amount of the shampoo composition can be applied to the hair or skin, which has been wetted with water, and then the composition can be rinsed off. Effective amounts can generally range from about 1 g to about 50 g, and from about 1 g to about 20 g. Application to the hair typically includes working the composition through the hair such that most or all of the hair is contacted with the composition.

A method for treating the hair or skin can include the steps of: (a) wetting the hair or skin with water; (b) applying an effective amount of the shampoo composition to the hair or skin, and (c) rinsing the applied areas of skin or hair with water. These steps can be repeated as many times as desired to achieve the desired cleansing and conditioning benefit.

A shampoo composition as described herein can be used to treat damaged hair. Damaged hair can include hair permed hair, oxidatively colored hair, and mechanically damaged hair.

The shampoo compositions can be used as liquids, solids, semi-solids, flakes, gels, in a pressurized container with a propellant added, or used in a pump spray form. The viscosity of the product may be selected to accommodate the form desired.

TEST METHODS

A. Cone/Plate Viscosity Measurement

The viscosities of the examples are measured by a Cone/Plate Controlled Stress Brookfield Rheometer R/S Plus, by Brookfield Engineering Laboratories, Stoughton, MA. The cone used (Spindle C-75-1) has a diameter of 75 mm and 1° angle. The viscosity is determined using a steady state flow experiment at constant shear rate of 2 $s^{-1}$ and at

temperature of 26.5 °C. The sample size is 2.5 ml and the total measurement reading time is 3 minutes.

B. pH Method

First, calibrate the Mettler Toledo Seven Compact pH meter. Do this by turning on the pH meter and waiting for 30 seconds. Then take the electrode out of the storage solution, rinse the electrode with distilled water, and carefully wipe the electrode with a scientific cleaning wipe, such as a Kimwipe®. Submerse the electrode in the pH 4 buffer and press the calibrate button. Wait until the pH icon stops flashing and press the calibrate button a second time. Rinse the electrode with distilled water and carefully wipe the electrode with a scientific cleaning wipe. Then submerse the electrode into the pH 7 buffer and press the calibrate button a second time. Wait until the pH icon stops flashing and press the calibrate button a third time. Rinse the electrode with distilled water and carefully wipe the electrode with a scientific cleaning wipe. Then submerse the electrode into the pH 10 buffer and press the calibrate button a third time. Wait until the pH icon stops flashing and press the measure button. Rinse the electrode with distilled water and carefully wipe with a scientific cleaning wipe.

Submerse the electrode into the testing sample and press the read button. Wait until the pH icon stops flashing and record the value.

C. Appearance Method

Following batch completion, first transfer batch to storage container. Second, sample batch in a glass vial. Third, visually inspect the sample. If the background can be seen distinctly record the appearance as transparent. If the background can be seen but distorted or hazy, record as semitransparent. If the background cannot be seen record as opaque.

D. Phase Stability Method

Following batch completion, first transfer batch to storage container. Second, sample batch in a glass vial. Third, visually inspect the sample. If sample is homogeneous record as one phase. If sample has two or more distinct phases record as phase separated including number of phases present. Distinct phases are visually different (changes in hazy, color, texture). These distinct phases either settle to the bottom, on top or are suspended.

E. Hair Switch Evaluation

This method describes how to evaluate performance of shampoo composition on hair switches. First, wet hair for 15 seconds. Second, apply shampoo to hair switch; 0.1g of shampoo per gram of hair. Then assess different attributes of shampoo performance on the hair switches throughout wash. Move hands up and down hair switch for 30 seconds. During these 30 seconds the following are assessed.

1.) Speed to Lather- Assessment of how quickly lather is generated (Scale: 0=Slow to 10=Fast)
2.) Lather Amount - Visual assessment of how much lather is generated (Scale: 0=Low to 10=High)

Next, run water over switch to begin rinsing. Rinse for 30 seconds. During the rinse assess the Rinse Feel/Rinse Count Drag. As soon as wetting begins, stroke the hair from top to bottom with non-dominant hand between the thumb and two fingers with medium pressure. Count the number of strokes until drag/skip is felt in the middle portion of the hair switch consistently for 2 consecutive strokes. This number of strokes is recorded as the Rinse Count Drag. The strokes should be at a rate of 1 stroke per second to a total of 20 strokes (Scale: 1= quick rinse/clean feel to 20 = slow rinse/dirty feel).

After the 30 second rinse stroke the hair from top to bottom with non-dominant hand between the thumb and two fingers with medium pressure to remove access water. Repeat stroke of hair switch and assess the clean feel of the hair. This is recorded as the Post Rinse - Clean Feel (Scale: 0=Low/Dirty to 10 = High/Clean).

F. Puff Lather Method

First, start water to allow it to reach desired temperature 37.5-38 C. Next, fluff the puff and wet it under the water. Apply the shampoo in a circular motion over top of the puff. Then move puff with lathered product overtop of a beaker. Next squeeze puff in a half turn forward direction 10 times. Repeat with squeeze and half turn in a backward direction 10 times. Lastly, squeeze puff to get all remaining lather out. Measure the amount of lather generated in the beaker.

G. Cylinder Lather Method - Lather Volume

Obtain 100 ml of Water in a 1,000 ml graduated cylinder. Then add 0.5g of shampoo. The graduated cylinder is on a rotating apparatus. Rotate the cylinder for 25 complete revolutions at a rate of about 10 revolutions per 18 seconds to create a lather and stop in a level, vertical position. A timer is set to allow 15 seconds for drainage. After 15 seconds, the lather volume is measured by recording the lather height to the nearest 10 ml mark (including any water that has drained to the bottom on top of which the lather is floating).

H. Kruss Lather Method

KRUSS Dynamic Foam Analyzer is used to evaluate lather. Add shampoo and water into device 1(shampoo) : 9 (water) dilution. Air going through the chamber generates lather. The speed to lather, lather volume, and bubble size are recorded.

NON-LIMITING EXAMPLES

The shampoo compositions illustrated in the following Examples illustrate specific embodiments of the shampoo compositions described herein but are not intended to be limiting thereof. Other modifications can be undertaken by the skilled artisan without departing from the spirit and scope of this invention. These exemplified embodiments of the shampoo composition provide suitable cleaning benefits to hair without the use of a harsh sulfate-based surfactant.

The shampoo compositions illustrated in the following Examples are prepared by conventional formulation and mixing methods, an example of which is set forth below. All exemplified amounts are listed as weight percent's and exclude minor materials such as diluents, preservatives, color solutions, imagery ingredients, botanicals, and so forth, unless otherwise specified. All percentages are based on weight unless otherwise specified.

Table 1: Examples of Shampoo Compositions

| | Ex.1 | Ex. 2 | Ex.3 | Ex.4 | Ex.5 | Ex.6 | Ex.7 | Ex.8 |
|---|---|---|---|---|---|---|---|---|
| Phase Stability | One phase | One phase | One phase | One phase | One phase | One phase | one phase | one phase |
| Appearance | semitransparent | semitransparent | semitransparent | semitransparent | semitransparent | semitransparent | transparent | transparent |
| pH | 5.28 | 5.07 | 5.40 | 5.45 | 5.51 | 5.34 | 4.53 | 5.62 |
| Viscosity (cps) | 1128 | 1045 | 3411 | 397 | 3732 | 427 | 505 | 659 |
| Decyl Glucoside[1] | 12 | | | | | | | |
| Decyl Glucoside [2] | | 8 | 12 | 12 | 12 | 12 | 8 | 20 |
| Cameilla Sinensis[3] | 0.005 | | | | | | | |
| Guar (3196) [4] | | | 0.95 | 0.50 | | | | |
| Guar (Dehyqart) [5] | | | | | 0.95 | 0.50 | | |
| Guar (Excel) [6] | 0.95 | 1.00 | | | | | 0.80 | 0.80 |
| Sodium Benzoate[7] | 0.60 | 0.60 | 0.60 | 0.60 | 0.60 | 0.60 | 0.60 | 0.60 |
| Citric Acid[8] | 0.40 | 0.40 | 0.40 | 0.40 | 0.40 | 0.40 | 0.40 | 0.40 |
| Fragrance | 0.70 | 0.65 | | | | 0.65 | 0.65 | 0.65 |
| Water | Q.S. | Q.S. | Q.S. | Q.S. | Q. S. | Q.S. | Q.S. | Q.S. |

Table 2: Comparative Examples of Shampoo Compositions

| Comparative Example Table: | | | | | | | | | |
|---|---|---|---|---|---|---|---|---|---|
| | Comp.1 | Comp. 2 | Comp. 3 | Comp. 4 | Comp. 5 | Comp.6 | Comp.7 | Comp.8 | Comp.9 |
| Phase Stability | One phase | One phase | One phase | One phase | One phase | One phase | One phase | One phase | Two phase |
| Appearance | transparent | transparent | transparent | transparent | transparent | transparent | transparent | transparent | semitransparent |
| pH | 5.32 | 5.12 | 5.11 | 5.08 | 5.30 | 5.49 | 5.07 | 5.02 | 5.40 |
| Viscosity (cps) | 3925 cps | 5000 cps | 2971 cps | 259 cps | too low to read | too low to read | too low to read | 15 cps | n/a |
| Decyl Glucoside [1] | 12 | | | | | | | | |
| Decyl Glucoside[2] | | 12 | 12 | 12 | 12 | 12 | 12 | 12 | 12 |
| Cameilla Sinensis [3] | 0.005 | | | | | | | | |
| Xanthan Gum [9] | 0.60 | 0.80 | | 0.20 | | | | | |
| Sclerotium Gum [10] | | | 0.75 | | | | | | |
| Guar (C500) [11] | | | | | 0.95 | | | | |
| Guar (Optima) [12] | | | | | | 0.95 | | | |
| Guar (3196) [4] | | | | | | | 0.25 | | |
| Guar (Excel) [6] | | | | | | | | | 1.20 |
| Polyquarternium-7[13] | | | | | | | | 1.00 | |
| Sodium Benzoate[7] | 0.60 | 0.60 | 0.60 | 0.60 | 0.60 | 0.60 | 0.60 | 0.60 | 0.60 |
| Citric Acid[8] | 0.40 | 0.40 | 0.40 | 0.40 | 0.40 | 0.40 | 0.40 | 0.40 | 0.40 |
| Fragrance | 0.50 | 0.65 | 0.65 | 0.65 | | | 0.65 | 0.65 | 0.70 |
| Water | Q.S. | Q.S. | Q.S. | Q.S. | Q. S. | Q. S. | Q.S. | Q.S. | Q.S. |

Suppliers for Examples in Table 1 to Table 2

1. Decyl Glucoside, Plantacare 2000 available from BASF®
2. Decyl Glucoside, Plantaren 2000 available from BASF®
3. Camellia Sinensis, Natpure Xtra Green Tea OP30 available from Sensient®
4. Guar hydroxypropyltrimonium chloride, N-Hance 3196 available from Ashland®
5. Guar hydroxypropyltrimonium chloride, Dehyquart Guar HP available from BASF®
6. Guar hydroxypropyltrimonium chloride, Jaguar Excel available from Solvay®
7. Sodium Benzoate, available from Kalama®
8. Citric Acid, available from Archer Daniels Midland®
9. Xanthan Gum, Keltrol LAX-T available from CP Kelco®
10. Sclerotium Gum, Amigum ER available from Alban Muller®
11. Guar hydroxypropyltrimonium chloride, Jaguar C500 available from Solvay®
12. Guar hydroxypropyltrimonium chloride, Jaguar Optima available from Solvay®
13. Polyquaternium 7, Merquat available by Lubrizol®

As can be seen in the data for the Examples and Comparative Examples, the Comparative Examples #1-4 do not contain cationic guar. Although they are able to meet viscosity target, they do not provide the desired moisturizing performance profile for the consumer. Comparative Examples 5-9 contain cationic guar having a weight average molecular weight of about 500,000, and these Comparative Examples do not meet the viscosity target for ease of consumer use.

It will be appreciated that other modifications of the present disclosure are within the skill of those in the hair care formulation art can be undertaken without departing from the spirit and scope of this invention. All parts, percentages, and ratios herein are by weight unless otherwise specified. Some components may come from suppliers as dilute solutions. The levels given reflect the weight percent of the active material, unless otherwise specified. A level of perfume and/or preservatives may also be included in the following examples.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests, or discloses any such invention. Further, to the extent that any meaning or definition of a term in this document conflicts with any meaning or definition of the same term in a document incorporated by reference, the meaning or definition assigned to that term in this document shall govern.

CLAIMS OF PARENT EUROPEAN PATENT APPLICATION 21 730 358.5

1. A shampoo composition comprising:

   a) from about 8 wt% to about 35 wt% of decyl glucoside;
   b) less than 1 wt% surfactant selected from sodium alkyl sulfate, sodium cocoyl isethionate, sodium lauroyl sarcosinate, cocamidopropyl betaine, sodium lauroamphoacetate, cetyltrimethylammonium chloride, behenyltrimethylammonium chloride and the mixture thereof;
   c) from about 0.4 wt% to about 1.2 wt% of cationic guar having a weight average molecular weight of from about 800,000/mol to about 5,000,000 g/mol and a charge density of from about 0.1 meg/g to about 2.5meg/g;

   wherein the shampoo composition has a viscosity from about 300cps to 8,000cps.

2. The shampoo composition according to any preceding claims, comprising about from about 8 wt% to about 30 wt% of the decyl glucoside.
3. The shampoo composition according to any preceding claims, comprising about from about 8 wt% to about 25 wt% of the decyl glucoside, preferably comprising about from about 7 wt% to about 20 wt% of the decyl glucoside.
4. The shampoo composition according to any preceding claims, wherein the composition further comprises a

polyglucoside selected from the group consisting of lauryl glucoside, octyl glucoside, caprylyl glucoside, caprylyl/-capryl glucoside, undecyl glucoside, coco glucoside, myristyl glucoside, hexadeyl glucoside, octadecyl glucoside, arachidyl glucoside, cetyl glucoside, cetearyl glucoside, isostearyl glucoside, and mixtures thereof

5. The shampoo composition according to any preceding claims, wherein the non-ionic surfactant is a decyl glucoside having the structure :

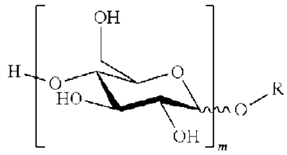

in which "R" is an alkyl or alkenyl group having 10 carbons, and "m" degree of polymerization is 1.

6. The shampoo composition according to any preceding claims, wherein the cationic guar has a weight average molecular weight of from about 800,000 g/mol to about 3,000,000 g/mol.

7. The shampoo composition according to any preceding claims, wherein the cationic guar has a weight average molecular weight of from about 900,000 g/mol to about 3,000,000 g/mol.

8. The shampoo composition according to any preceding claims, wherein the cationic guar has a weight average molecular weight of from about 800,000 g/mol to about 2,500,000 g/mol.

9. The shampoo composition according to any preceding claims, wherein the cation guar has a weight average molecular weight of from about 900,000 g/mol to about 2,500,000 g/mol.

10. The shampoo composition according to any preceding claims, wherein the charge density is from about 0.3 meq/g to about 1.9 meg/g.

11. The shampoo composition according to any preceding claims, wherein the charge density is from about 0.4 meq/g to about 1.8 meg/g, preferably wherein the charge density is from about 0.5 meq/g to about 1.7 meg/g, even more preferably wherein the charge density is from about 0.7 meq/g to about 1.25 meg/g.

12. The shampoo composition according to any preceding claims, wherein the composition further comprises a material selected from the group consisting of tea extracts, grape seed extracts, safflower oil, jojoba oil, argan oil, and combinations thereof.

13. The shampoo composition according to any preceding claims, wherein the cationic guar is guar hydroxylpropyltrimonium chloride.

14. The shampoo composition according to any preceding claims, wherein the composition further comprises an antimicrobial agent selected from the groups consisting of: azoles, climbazole, ketoconazole, itraconazole, econazole, elubiol, hydroxy pyridones, piroctone olamine, ciclopirox, rilopirox, MEA-Hydroxyoctyloxypyridinon, kerolytic agents, salicylic acid, hydroxy acids, strobilurins, azoxystrobin, metal chelators, 1,10-phenanthroline, and combinations thereof.

## Claims

**1.** A shampoo composition comprising:

a) from 8 wt.% to 35 wt.% of decyl glucoside;
b) less than 1 wt.% surfactant selected from sodium alkyl sulfate, sodium cocoyl isethionate, sodium lauroyl sarcosinate, cocamidopropyl betaine, sodium lauroamphoacetate, cetyltrimethylammonium chloride, behenyltrimethylammonium chloride and the mixture thereof;
c) from 0.4 wt.% to 1.0 wt.%, or from 0.8 wt.% to 1.0 wt.% of cationic guar having a weight average molecular weight of from 800,000 g/mol to 5,000,000 g/mol as measured by gel permeation chromatography and a charge density of from 0.1 meq/g to 2.5 meq/g;

wherein the shampoo composition has a viscosity from 0.3 Pa.s (300 cps) to 8.0 Pa.s (8,000 cps) as measured with a Cone/Plate Controlled Stress Brookfield Rheometer R/S Plus, by Brookfield Engineering Laboratories, Stoughton, MA, wherein the cone used is a Spindle C-75-1 having a diameter of 75 mm and 1° angle, wherein the viscosity is determined using a steady state flow experiment at constant shear rate of 2 $s^{-1}$ and at temperature of 26.5°C, wherein a sample size is 2.5 ml and a total measurement reading time is 3 minutes.

**2.** The shampoo composition according to claim 1, comprising from 8 wt.% to 30 wt.% of the decyl glucoside.

3. The shampoo composition according to any preceding claims, comprising from 8 wt.% to 25 wt.% of the decyl glucoside, preferably comprising from 7 wt.% to 20 wt.% of the decyl glucoside.

4. The shampoo composition according to any preceding claims, wherein the composition further comprises a polyglucoside selected from the group consisting of lauryl glucoside, octyl glucoside, caprylyl glucoside, caprylyl/-capryl glucoside, undecyl glucoside, coco glucoside, myristyl glucoside, hexadecyl glucoside, octadecyl glucoside, arachidyl glucoside, cetyl glucoside, cetearyl glucoside, isostearyl glucoside, and mixtures thereof.

5. The shampoo composition according to any preceding claims, wherein the decyl glucoside has the structure :

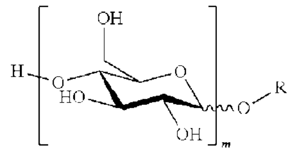

in which "R" is an alkyl or alkenyl group having 10 carbons, and "m" degree of polymerization is 1.

6. The shampoo composition according to any preceding claims, wherein the cationic guar has a weight average molecular weight of from 800,000 g/mol to 3,000,000 g/mol.

7. The shampoo composition according to any preceding claims, wherein the cationic guar has a weight average molecular weight of from 900,000 g/mol to 3,000,000 g/mol.

8. The shampoo composition according to any preceding claims, wherein the cationic guar has a weight average molecular weight of from 800,000 g/mol to 2,500,000 g/mol.

9. The shampoo composition according to any preceding claims, wherein the cation guar has a weight average molecular weight of from 900,000 g/mol to 2,500,000 g/mol.

10. The shampoo composition according to any preceding claims, wherein the charge density is from 0.3 meq/g to 1.9 meq/g.

11. The shampoo composition according to any preceding claims, wherein the charge density is from 0.4 meq/g to 1.8 meq/g, preferably wherein the charge density is from 0.5 meq/g to 1.7 meq/g, even more preferably wherein the charge density is from 0.7 meq/g to 1.25 meq/g.

12. The shampoo composition according to any preceding claims, wherein the composition further comprises a material selected from the group consisting of tea extracts, grape seed extracts, safflower oil, jojoba oil, argan oil, and combinations thereof.

13. The shampoo composition according to any preceding claims, wherein the cationic guar is guar hydroxylpropyltrimonium chloride.

14. The shampoo composition according to any preceding claims, wherein the composition further comprises an anti-microbial agent selected from the groups consisting of: azoles, climbazole, ketoconazole, itraconazole, econazole, elubiol, hydroxy pyridones, piroctone olamine, ciclopirox, rilopirox, MEA-Hydroxyoctyloxypyridinon, kerolytic agents, salicylic acid, hydroxy acids, strobilurins, azoxystrobin, metal chelators, 1,10-phenanthroline, and combinations thereof.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4185087 A
- EP 0530974 A
- WO 2002010257 A
- US 2809971 A
- US 3236733 A
- US 3753196 A
- US 3761418 A
- US 4345080 A
- US 4323683 A
- US 4379753 A
- US 4470982 A
- US 2694668 A
- US 3152046 A
- US 4089945 A
- US 4885107 A